(19) Europäisches Patentamt
European Patent Office
Office européen des brevets

(11) Veröffentlichungsnummer: **0 106 797**
**B1**

# (12) EUROPÄISCHE PATENTSCHRIFT

(45) Veröffentlichungstag der Patentschrift: **02.10.85**

(51) Int. Cl.⁴: **C 07 C 25/18,** C 07 C 79/12 // C08K5/04

(21) Anmeldenummer: **83810410.7**

(22) Anmeldetag: **12.09.83**

(54) **Diaryljodosylsalze und Verfahren zu deren Herstellung.**

(30) Priorität: **18.09.82 GB 8226706**
**10.06.83 GB 8315925**

(43) Veröffentlichungstag der Anmeldung:
**25.04.84 Patentblatt 84/17**

(45) Bekanntmachung des Hinweises auf die Patenterteilung:
**02.10.85 Patentblatt 85/40**

(84) Benannte Vertragsstaaten:
**BE CH DE FR GB IT LI NL SE**

(56) Entgegenhaltungen:
**JOURNAL OF ORGANIC CHEMISTRY, Band 33, Nr. 7, Juli 1968, Seiten 2981-2984, Easton, USA F.M. BERINGER et al.: "Diaryliodosyl salts"**

(73) Patentinhaber: **CIBA-GEIGY AG, Postfach, CH-4002 Basel (CH)**

(72) Erfinder: **Irving, Edward, Dr., 41, Swaffham Road, Burwell Cambridge CB5 OAN (GB)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Beschreibung

Vorliegende Erfindung betrifft neue Diaryljodosylsalze, die als Katalysatoren für die Polymerisation kationisch polymerisierbarer Materialien verwendbar sind, sowie deren Herstellung.

Es ist wohlbekannt, daß gewisse Jodoniumsalze, wenn sie aktinischer Strahlung unterworfen werden, eine Art saure Verbindung freisetzen, die dann dazu verwendet werden kann, kationisch polymerisierbare Materialien wie Epoxidharze, Phenoplaste und Aminoplaste zu polymerisieren bzw. härten.

Beispielsweise beschreibt die britische Patentschrift Nr. 1 491 540 aromatische Jodoniumkomplexsalze der allgemeinen Formel

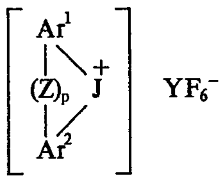

(I)

worin $Ar^1$ und $Ar^2$ gleich oder verschieden sein können und je für eine aromatische Gruppe mit 4 bis 20 Kohlenstoffatomen, wie eine gegebenenfalls substituierte Phenyl-, Thienyl-, Furanyl- oder Pyrazolylgruppe und Z für ein Sauerstoffatom, ein Schwefelatom oder eine Gruppe der Formel

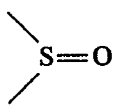 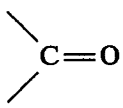 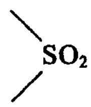 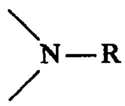

(worin R ein Wasserstoffatom, eine Alkylgruppe mit 1 bis 4 Kohlenstoffatomen oder eine Carbonsäureacylgruppe bedeutet), eine direkte Kohlenstoff-Kohlenstoffbindung oder eine Gruppe der Formel

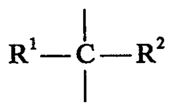

(worin $R^1$ und $R^2$ gleich oder verschieden sein können und je ein Wasserstoffatom, eine Alkylgruppe mit 1 bis 4 Kohlenstoffatomen oder eine Alkenylgruppe mit 2 bis 4 Kohlenstoffatomen bedeuten) stehen, p null oder 1 ist und Y ein Phosphor-, Arsen- oder Antimonatom darstellt.

In der britischen Patentschrift Nr. 1 516 352 sind Salze der Formel

$$\left[(R^3)_a(R^4)_b\ \overset{+}{hal}\right]\left[YF_6\right]^- \qquad (II)$$

worin Y die oben angegebene Bedeutung hat, $R^3$ einen einwertigen aromatischen organischen Rest, $R^4$ einen zweiwertigen aromatischen organischen Rest und »hal« einen Halogenrest bedeuten und a null und b 1 bzw. a 2 und b null sind, beschrieben.

Aus der britischen Patentschrift Nr. 1 539 192 sind photopolymerisierbare Zusammensetzungen bekannt, die aus mindestens einem säurepolymerisierbaren oder säurehärtbaren Material und als Photosensibilisator mindestens einem Jodoniumsalz der Formel

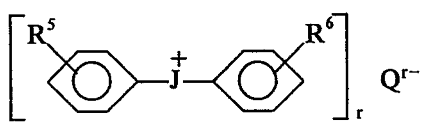

(III)

worin r 1 oder 2 ist, $R^5$ und $R^6$ gleich oder verschieden sein können und je für ein Wasserstoff- oder Halogenatom, eine Nitrogruppe, eine gegebenenfalls substituierte Kohlenwasserstoffgruppe oder eine heterocyclische Gruppe stehen und $Q^{r-}$ ein sich von einer zur Polymerisation oder Härtung des säurepolymerisierbaren oder säurehärtbaren Materials fähigen Säure ableitendes Anion darstellt, bestehen.

In einem Artikel von F. M. Beringer und P. Bodlaender, J. Org. Chem., 1968, 33, 2981-4, sind verschiedene Diaryljodosylsalze und deren Herstellung beschrieben. Die isolierten Salze waren sämtlich Acetate, Trifluoracetate, Jodate, Chloride, Bromide oder Fluoride. Ein Jodoxybenzol wird in Gegenwart von Hydroxydionen einer Selbstkondensation zu einem Diphenyljodosylhydroxyd unterzogen, das mit Essig-, Jod- oder Trifluoressigsäure neutralisiert wird. Die übrigen Salze wurden daraus durch doppelte Umsetzung hergestellt. Typische solche Salze sind Diphenyljodosylacetat, -trifluoracetat, -fluorid, -chlorid und -bromid sowie Di-(4-methylphenyl)-jodosyljodat. Dieser Artikel gibt keinen Hinweis auf das Verhalten solcher Salze, wenn sie aktinischer Strahlung unterworfen werden, und gibt keine

mögliche Verwendung für diese an.

Überraschenderweise wurde nun gefunden, daß gewisse neuartige Jodosylsalze als photoaktivierbare Härtungs- oder Polymerisationsmittel für kationisch polymerisierbare Materialien verwendbar sind; der Stand der Technik gibt keinen Hinweis darauf, daß Jodosylsalze eine solche Aktivität aufweisen würden.

Gegenstand dieser Erfindung sind dementsprechend neuartige Diaryljodosylsalze der allgemeinen Formel

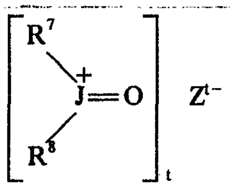

(IV)

worin $R^7$ und $R^8$ gleich oder verschieden sein können und je einen einwertigen aromatischen Rest mit 6 bis 22 Kohlenstoffatomen darstellen, $Z^{t-}$ für ein t-wertiges Anion der Formel $MX_n^-$ oder $R^9SO_3^-$ oder ein t-wertiges Anion einer halogenfreien anorganischen Sauerstoffsäure steht, t 1, 2 oder 3 ist, M ein Metall- oder Metalloidatom darstellt, X ein Halogenatom, vorzugsweise Fluor oder Chlor, bedeutet, n 4, 5 oder 6 und um eins größer als die Wertigkeit von M ist, mit der Maßgabe daß, wenn M für Antimon steht, n 6 ist und wenn fünf der Symbole X je Fluor bedeuten, das sechste X für ein Fluoratom oder eine Hydroxygruppe steht, und $R^9$ eine einwertige aliphatische oder aromatische Gruppe mit 1 bis 20 Kohlenstoffatomen darstellt.

Vorzugsweise bedeutet M ein Antimon-, Wismut-, Zinn- oder besonders bevorzugt Bor-, Eisen-, Arsen- oder Phosphoratom. Das Anion $MX_n^-$ stellt daher vorzugsweise ein Pentafluorohydroxoantimonat-, Hexachloroantimonat-, Hexafluoroantimonat- oder besonders bevorzugt Tetrafluoroborat-, Tetrachloroferrat-, Hexafluoroarsenat- oder Hexafluorophosphatanion dar.

Vorzugsweise steht $R^9$ für eine aromatische, gegebenenfalls durch Alkylgruppen mit 1 bis 4 Kohlenstoffatomen oder ein oder mehrere Halogenatome substituierte aromatische Gruppe mit 6 bis 12 Kohlenstoffatomen, insbesondere Phenyl- oder Toluylgruppe, oder eine gegebenenfalls halogensubstituierte aliphatische Gruppe mit 1 bis 4 Kohlenstoffatomen, insbesondere eine Methyl- oder Trifluormethylgruppe.

Als halogenfreie anorganische Sauerstoffsäureanionen werden Phosphat-, einschließlich Orthophosphaten und Hydrogenphosphaten, Sulfat- und Hydrogensulfatanionen bevorzugt.

Ferner ist es vorzuziehen, daß die Gruppe $R^7$ und $R^8$ gleich sind und für gegebenenfalls durch unter Alkoxygruppen mit 1 bis 4 Kohlenstoffatomen, Phenylgruppen und insbesondere Alkylgruppen mit 1 bis 4 Kohlenstoffatomen, Nitrogruppen und Halogenatomen ausgewählte Gruppen einfach oder zweifach substituierte Phenyl- oder Naphthylgruppen stehen.

Die Diaryljodosylsalze der Formel IV sind durch Umsetzung eines Diaryljodosylhydroxyds der Formel

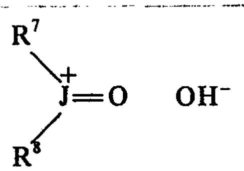

(V)

worin $R^7$ und $R^8$ die oben angegebenen Bedeutungen haben, mit einer Säure der Formel $H_tZ$ (falls solche Säuren existieren), nämlich der Säure, von der sich $Z^{t-}$ ableitet, oder mit einem Alkali- oder Ammoniumsalz einer solchen Säure, wobei $Z^{t-}$ die oben angegebene Bedeutung hat, herstellbar. Diese Umsetzung wird vorzugsweise in Wasser oder einem wäßrigen organischen Lösungsmittel bei oder unterhalb Raumtemperatur, vorzugsweise bei 0° bis 20° C, durchgeführt.

In Abänderung dieser Verfahrensweise kann man das Hydroxyd der Formel V durch das entsprechende Carbonat der Formel

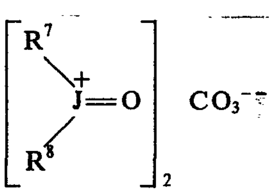

(VI)

ersetzen, doch wird dabei die Säure der Formel $H_tZ$ verwendet.

Salze der Formel IV lassen sich ferner durch Umsetzung eines Diaryljodosylacetats der Formel VII,

eines Trifluoracetats der Formel VIII oder eines Diaryljodosylhalogenids der Formel IX

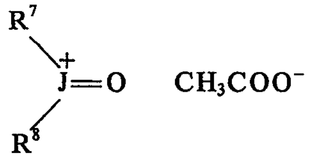 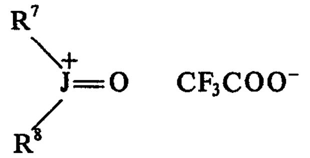 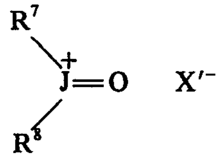

(VII) (VIII) (IX)

mit einem Anion der Formel $Z^{t-}$ herstellen, wobei $R^7$, $R^8$, $Z^-$ und t die oben angegebenen Bedeutungen haben und X′ ein Halogenatom darstellt. Diese Umsetzung wird vorzugsweise mit einem Ammonium- oder Alkalisalz des Anions, insbesondere dem Natrium- oder Kaliumsalz, oder in manchen Fällen dem Silbersalz in Wasser oder einem wäßrigen organischen Lösungsmittel bei Raumtemperatur oder unter mildem Erhitzen bis auf etwa 100° C durchgeführt.

Erfolgt eine solche doppelte Umsetzung mit einer wäßrigen Lösung eines Alkalihexafluoroantimonats, so ist das erhaltene Salz der Formel V wegen Hydrolyse das Pentafluorohydroxoantimonat; um das entsprechende Hexafluoroantimonat zu erhalten, muß man das Alkalihexafluoroantimonat als Feststoff zusetzen.

Die bei den oben beschriebenen Verfahren als Ausgangsstoffe verwendeten Diaryljodosylverbindungen der Formeln V bis IX werden zweckmäßig nach F. M. Beringer und P. Bodlaender, a. a.O., aus Jodoxyarenen hergestellt, die man nach J. G. Sharefkin und H. Saltzman, Org. Syn., 1963, 43, 665-7, wo auf Beringer und Bodlaender Bezug genommen wird, erhalten kann. Ein oder zwei Jodoxyarene der Formeln X und XI

| | |
|---|---|
| $R^7JO_2$ | $R^8JO_2$ |
| (X) | (XI) |

worin $R^7$ und $R^8$ die oben angegebenen Bedeutungen haben, werden mit einem Alkalihydroxyd behandelt, um das Diaryljodosylhydroxyd der Formel V zu bilden. Bei dessen Behandlung mit Kohlendioxyd erhält man das Carbonat der Formel VI, und Behandlung mit Essigsäure, Trifluoressigsäure bzw. einer Halogenwasserstoffsäure ergibt das Acetat der Formel VII, Trifluoracetat der Formel VIII bzw. Halogenid der Formel IX.

Wie oben erwähnt sind die erfindungsgemäßen Salze als Photoinitiatoren zur Polymerisation kationisch polymerisierbarer Materialien verwendbar. Aus einem Diaryljodosylsalz der Formel IV und einem kationisch polymerisierbaren Material bestehende Zusammensetzungen sind üblicherweise für Strahlung mit einer Wellenlänge im Bereich 200 bis 600 nm, d. h. über das ganze sichtbare und ultraviolette Spektralgebiet, empfindlich. Belichtung für verhältnismäßig kurze Zeit, insbesondere im ultravioletten Gebiet, ergibt ein Polymer mit wünschenswerten Eigenschaften.

Spezielle Beispiele für erfindungsgemäße Diaryljodosylsalze sind unter anderem: Bis-(4-methoxyphenyl)-jodosylhexafluorophosphat, Bis-(4-fluorophenyl)-jodosylhexafluorophosphat, Diphenyljodosyl-hexafluorophosphat, Bis-(4-methylphenyl)-jodosyl-hexafluorophosphat, Bis-(4-biphenyl)-jodosylhexafluorophosphat, Bis-(2,4-dichlorphenyl)-jodosyl-hexafluorophosphat, Bis-(1-naphtyl)-jodosylhexafluorophosphat, Bis-(4-isopropylphenyl)-jodosyl-hexafluorophosphat, Bis-(3-nitrophenyl)-jodosyl-hexafluorophosphat, die entsprechenden Tetrafluoroborate, Hexafluoroarsenate, Hexachloroantimonate und Hexafluoroantimonate, Diphenyljodosyl-4-toluolsulfonat, Diphenyljodosyl-tetrachloroferrat, Diphenyljodosyl-orthophosphat und Diphenyljodosyl-sulfat.

Die Erfindung wird durch die nachfolgenden Beispiele erläutert.

Die in den Beispielen verwendeten Jodoxyarene werden nach J. G. Sharefkin und H. Saltzman, a. a. O., hergestellt.

## Beispiel 1

Man löst Diphenyljodosylacetatmonohydrat (1,87 g; nach F. M. Beringer und P. Bodlaender, a. a. O., hergestellt) in siedendem Wasser (25 ml), behandelt mit Entfärbungskohle und filtriert heiß ab. Das Filtrat wird langsam mit gesättigter wäßriger Kaliumhexafluorophosphatlösung versetzt, bis kein Niederschlag mehr auftritt. Dann wird abgekühlt und filtriert. Der Rückstand wird im Vakuum bei Raumtemperatur über Phosphorpentoxyd getrocknet, wobei man Diphenyljodosylhexafluorophosphat (0,87 g) vom Schmelzpunkt 120—130° C (Zers.) erhält.

## Beispiel 2

Man gibt Jodoxybenzol (3,37 g) unter Rühren zu 1 n-Natronlauge bei 0° C. Nach 2 Stunden filtriert man und versetzt mit einer Lösung von Kaliumhexafluorophosphat (1,32 g) in Wasser (5 ml), wobei sich

sofort ein flockiger Niederschlag bildet. Dieser wird abfiltriert, mit eiskaltem Wasser gewaschen, über Phosphorpentoxyd getrocknet und aus Chloroform/Petroläther (Siedebereich 40°—60° C) umkristallisiert, wobei man Diphenyljodosylhexafluorophosphat (0,18 g) vom Schmelzpunkt 130° C (Zers.) erhält.

### Beispiel 3

Man löst Diphenyljodosyltrifluoracetat (4,1 g; nach F. M. Beringer und P. Bodlaender, a. a. O., hergestellt) durch portionsweise Zugabe vollständig in siedendem Wasser und behandelt die Lösung dann mit Aktivkohle und filtriert heiß ab. Das heiße Filtrat wird mit einer gesättigten wäßrigen Lösung von 1,84 g Kaliumhexafluorophosphat versetzt und die entstehende klare Lösung abgekühlt. Es bildet sich ein weißer Niederschlag, der abfiltriert wird, was Diphenyljodosylhexafluorophosphat (2,72 g) vom Schmelzpunkt 135—136° C (Zers.) ergibt. (Gefunden: C 33,30; H 2,36; F 26,04; J 29,18; P 7,13; für $C_{12}H_{10}F_6JOP$ berechnet C 32,60; H 2,28; F 25,79; J 28,71; P 7,01%).

Die Spektralanalyse zeigt:
Infrarot (KBr-Preßling) $\nu_{max}$ bei 3100, 3065, 1580, 1565, 1470, 1445, 1010, 995, 990, 840 (breit), 755, 745, 740, 685 und 650 $cm^{-1}$.
Ultraviolett $\lambda_{max}$ in $CHCl_3$ 227 nm.
N. M. R. (in deuteriertem Aceton) $\delta$ bei 8,50—8,20 (m) und 7,85—7,40 (m).

Die obigen Zahlen stehen mit der dem Produkt zugeschriebenen Konstitution als Diphenyljodosylhexafluorophosphat in Einklang.

### Beispiel 4

Man gibt Jodoxybenzol (35,4 g) zu 1 n-Natronlauge (300 ml) unter Rühren und Abkühlung auf 0° C. Nach 2 Stunden wird der Niederschlag durch Filtrieren entfernt und das Filtrat mit Kohlendioxyd behandelt, bis es neutral wird.

Ein Teil dieser neutralisierten Lösung (100 ml) wird unter Rühren langsam mit Fluoroborsäure (40%) versetzt, bis die Kohlendioxydentwicklung zum Stillstand kommt; dann wird eine weitere Menge dieser Säure (2 ml) zugesetzt. Der gebildete Niederschlag wird abfiltriert, mit eiskaltem Wasser gewaschen und getrocknet, wobei man Diphenyljodosyl-tetrafluoroborat (2,4 g) vom Schmelzpunkt 110—120° C (Zers.) erhält.

Die Infrarotspektralanalyse (KBr-Preßling) zeigt $\nu_{max}$ bei 3080, 3050, 1465, 1445, 1060 (breit), 985, 740 und 680 $cm^{-1}$.

### Beispiel 5

Man löst Bis-(4-methylphenyl)-jodosyl-trifluoracetat (1,16 g; nach F. M. Beringer und P. Bodlaender, a. a. O., hergestellt) in siedendem Wasser (90 ml), filtriert und versetzt mit einer gesättigten wäßrigen Lösung von 0,49 g Kaliumhexafluorophosphat. Der weiße Niederschlag wird abfiltriert und getrocknet, was Bis-(4-methylphenyl)-jodosyl-hexafluorophosphat (0,68 g) vom Schmelzpunkt 125° C (Zers.) ergibt.

### Beispiel 6

Man gibt 4-Jodoxytoluol (12,5 g) unter Rühren zu auf 0° C gekühlte 2 n-Natronlauge (100 ml). Nach 2 Stunden wird der Niederschlag durch Filtrieren entfernt. Das Filtrat versetzt man bei 0° C unter Rühren mit einer gekühlten 10%igen Hexafluorophosphorsäurelösung. Der gebildete weiße Niederschlag wird durch Filtrieren gewonnen, mit Wasser gewaschen und an der Luft getrocknet, was Bis-(4-methylphenyl)-jodosyl-hexafluorophosphat (7,6 g) vom Schmelzpunkt 127—128° C ergibt.

Eine zur Spektralanalyse eingereichte Probe liefert die folgenden Ergebnisse:
Infrarot (KBr-Preßling) $\nu_{max}$ bei 1475, 1180, 995, 848, 801, 745,
$^1$H-n.m.r. (in deuteriertem Aceton) $\delta$ bei 7,65 (8H, d von d) und 2,4 (6H, s)
U.V. $\lambda_{max}$ in $CHCl_3$ 238 nm.

### Beispiel 7

Unter Verwendung von 4-Isopropyljodoxybenzol (8,3 g) als Ausgangsstoff stellt man Bis-(4-isopropylphenyl)-jodosyl-trifluoracetat auf ähnliche Weise wie nach F. M. Beringer und P. Bodlaender, a. a. O., her. Das Trifluoracetat wird in siedendem Wasser gelöst. Dann versetzt man mit Kaliumhexafluorophosphat (2-molarer Überschuß) in Wasser und läßt die Lösung abkühlen. Die gebildeten Kristalle werden durch Filtrieren gewonnen und an der Luft getrocknet, was Bis-(4-isopropylphenyl)-jodosyl-hexafluorophosphat (0,4 g) vom Schmelzpunkt 84° C liefert. Die spektroskopische Analyse zeigt:
I.R. (KBr-Preßling $\nu_{max}$ 2970, 1655, 1480, 1410, 992, 848
$^1$H-nmr ($d^6$-Aceton) $\delta$ bei 7,85 (8H, d von d), 2,75 (2H, m) und 1,2 (12H, d)
U.V. (EtOH) $\lambda_{max}$ 237.

Beispiel 8

Man stellt Bis-(4-fluorphenyl)-jodosyl-hexafluorophosphat analog wie Bis-(4-methylphenyl)-jodosyl-hexafluorophosphat in Beispiel 6 her. Aus 4-Fluorjodoxybenzol (10,2 g) erhält man eine Ausbeute von 0,5 g vom Schmelzpunkt 126° C (Zersetzung).

Infrarot- und magnetische Protonenresonanzspektroskopie der Verbindung ergibt folgende Analyse:
Infrarot (KBr-Preßling) $\nu_{max}$ bei 1580, 1480, 1242, 1165, 848, 730
$^1$H-nmr (in deuteriertem Aceton) $\delta$ bei 8,3 und 7,4, breite Multipletts.
U.V. $\lambda_{max}$ 225 nm, Schulter bei 258 nm.

Beispiel 9

Man löst Diphenyljodosyltrifluoracetat (1,2 g; nach F. M. Beringer und P. Bodlaender, a. a. O., hergestellt) in heißem Wasser, behandelt mit Aktivkohle und filtriert heiß. Das Filtrat versetzt man mit einer wäßrigen Lösung von Kaliumhexafluoroarsenat (0,7 g) und kühlt die erhaltene Lösung. Der gebildete weiße Niederschlag wird durch Filtrieren gewonnen und an der Luft getrocknet, was Diphenyljodosylhexafluoroarsenat (0,64 g) vom Schmelzpunkt 135—138° C (Zersetzung) ergibt. Die Spektralanalyse zeigt:
I.R. (KBr-Preßling) $\nu_{max}$ bei 1470, 1445, 1200, 1133, 989, 738 und 704.
$^1$H-nmr (in deuteriertem Aceton) $\delta$ bei 8,2 m und 7,65 m.

Beispiel 10

Man gibt Jodoxybenzol (11,8 g) unter Rühren bei 0° C zu 1-molarer Natronlauge. Nach 2 Stunden wird filtriert und das Filtrat bei 0° C mit Kohlendioxyd behandelt, bis es neutral wird. Unter kräftigem Rühren gibt man eine wäßrige 4-Toluolsulfonsäurelösung dazu, bis vollständige Freisetzung des Kohlendioxyds erreicht ist. Der gebildete Feststoff wird durch Filtrieren gewonnen, mit eiskaltem Wasser gewaschen und im Vakuum über Phosphorpentoxyd getrocknet, was Diphenyljodosyl-4-toluolsulfonat (7,75 g) vom Schmelzpunkt 102° C ergibt. Die Spektralanalyse zeigt:
Infrarot (KBr-Preßling) $\nu_{max}$ bei 1470, 1442, 1200, 1120, 1032, 1010, 740 und 685
$^1$H-nmr (in deuteriertem Aceton) $\delta$ bei 8,15 m, 7,5 m und 6,95 d, 14 Protonen im ganzen, und 2,25 s, 3H.
U.V. $\lambda_{max}$ in $H_2O$ 222 nm, Schulter bei 262 nm.

Beispiel 11

Man löst Diphenyljodosyltrifluoracetat (1 g) in siedendem Wasser. Die Lösung wird mit Aktivkohle behandelt und dann heiß filtriert. Nach Zugabe von Ammoniumtetrachloroferrat bildet sich ein weißer Niederschlag. Der Feststoff wird durch Filtrieren gewonnen und an der Luft getrocknet, was Diphenyljodosyl-tetrachloroferrat (0,44 g) vom Schmelzpunkt 164° C ergibt.

Beispiel 12

Man gibt Jodoxybenzol (11,8 g) unter Rühren bei 0° C zu 1 n-Natronlauge (100 ml). Man rührt noch weitere 1$^1/_2$ Stunden bei dieser Temperatur. Dann wird zur Entfernung des ausgefallenen Natriumjodats filtriert. Das Filtrat behandelt man mit 10%iger Orthophosphorsäurelösung, bis es sauer wird (pH 5). Kristallisation erfolgt durch Kühlung der Lösung auf 0° C über Nacht. Die Kristalle werden durch Filtrieren gewonnen und an der Luft getrocknet, was Diphenyljodosylorthophosphat (2,5 g) vom Schmelzpunkt 115—116° C (Zersetzung) liefert.

Beispiel 13

Diphenyljodosyl-sulfat wird analog wie Diphenyljodosyl-orthophosphat in Beispiel 12 hergestellt, jedoch unter Verwendung einer 10%igen Schwefelsäurelösung anstatt der Orthophosphorsäure. Bei

der Zugabe der Säure bildet sich ein gelber Niederschlag; dieser wird abfiltriert und der Feststoff gewonnen. Der gelbliche Feststoff ist Diphenyljodosyl-sulfat (2,0 g) vom Schmelzpunkt 126—128° C (Zersetzung).

Beispiel 14

Man gibt 3-Nitrojodoxybenzol (11,25 g) vom Schmelzpunkt 206° C (Zersetzung), in 75% Ausbeute nach J. G. Sharefkin und H. Saltzman, a. a. O. hergestellt, unter Rühren bei 0° C zu 1-molarer Natronlauge (80 ml). Nach 90 Minuten filtriert man, um das ausgefallene Natriumjodat zu entfernen. Das Filtrat wird mit Hexafluorophosphorsäurelösung (10%) versetzt. Mit der Erniedrigung des pH fällt das Jodosylsalz aus der Lösung aus. Die Säurezugabe wird beendet, wenn der pH ungefähr 5 erreicht. Der Niederschlag wird durch Filtrieren gewonnen und an der Luft getrocknet. Er stellt Bis-(3-nitrophenyl)-jodosyl-hexafluorophosphat (0,2 g) vom Schmelzpunkt 182° C (Zersetzung) dar.

Die nachfolgenden Beispiele erläutern die Verwendung der erfindungsgemäßen Salze als Photokatalysatoren für die Polymerisation kationisch polymerisierbarer Materialien. Die folgenden Harze werden in diesen Beispielen verwendet:

Harz I bedeutet den Diglycidyläther des 2,2-Bis-(4-hydroxyphenyl)-propans.
Harz II bedeutet 3,4-Epoxicyclohexancarbonsäure-3,4-epoxicyclohexylmethylester.
Harz III bedeutet ein aus Phenol und Formaldehyd hergestelltes Resol mit einem Phenol/Formaldehydmolverhältnis von 1 : 1,14, einer Viskosität von 0,7 Pa · s bei 25° C und einem Feststoffgehalt von 76%, mit 4-Toluolsulfonsäure neutralisiert.

Beispiel 15

Man vermischt das Harz (10 g) mit Diphenyljodosylhexafluorophosphat (0,3 g) und Aceton (0,15 ml) und streicht es als 10 µm dicken Überzug auf Weißblech. Der Überzug wird mit einer 80 W/cm-Mitteldruckquecksilberbogenlampe in 20 cm Abstand bestrahlt. Die verwendeten Harze, die Bestrahlungszeiten und die Eigenschaften der bestrahlten Überzüge sind in der nachfolgenden Tabelle angegeben.

Tabelle 1

| Harz | Bestrahlungszeit (Sekunden) | Bestrahlter Überzug |
|---|---|---|
| I | 1 | hart, klebfrei |
| II | 1 | hart, klebfrei |

Beispiel 16

Man vermischt Harz III (100 g) mit Diphenyljodosyl-4-toluolsulfonat (3 g) und streicht es auf Weißblech zu einem 6—8 µm dicken Überzug auf. Der Überzug wird mit einer 80 W/cm-Mitteldruckquecksilberbogenlampe in 20 cm Abstand bestrahlt, was nach 8 Sekunden einen klebfreien Film ergibt.

**Patentansprüche**

1. Diaryljodosylsalze der Formel

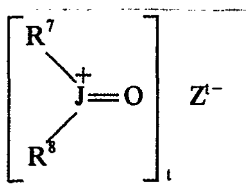

(IV)

worin $R^7$ und $R^8$ gleich oder verschieden sein können und je einen einwertigen aromatischen Rest mit 6 bis 22 Kohlenstoffatomen darstellen, $Z^{t-}$ für einen t-wertiges Anion der Formel $MX_n^-$ oder $R^9SO_3^-$ oder ein t-wertiges Anion einer halogenfreien anorganischen Sauerstoffsäure steht, t 1, 2 oder 3 ist, M ein Metall- oder Metalloidatom darstellt, X ein Halogenatom bedeutet, n 4, 5 oder 6 und um eins größer

als die Wertigkeit von M ist, mit der Maßgabe daß, wenn M für Antimon steht, n 6 ist und wenn fünf der Symbole X je Fluor bedeuten, ein X für ein Fluoratom oder eine Hydroxogruppe steht, und $R^9$ eine einwertige aliphatische oder aromatische Gruppe mit 1 bis 20 Kohlenstoffatomen darstellt.

2. Salze nach Anspruch 1, dadurch gekennzeichnet, daß $Z^{t-}$ für $MX_n^-$ steht, worin M ein Antimon-, Wismut-, Zinn-, Bor-, Eisen-, Arsen- oder Phosphoratom bedeutet.

3. Salze nach Anspruch 1 oder 2, dadurch gekennzeichnet, daß $Z^{t-}$ für $MX_n^-$ steht, worin X ein Fluor- oder Chloratom bedeutet.

4. Salze nach Anspruch 3, dadurch gekennzeichnet, daß $MX_n^-$ das Pentafluorohydroxoantimonat-, Hexachloroantimonat-, Hexafluoroantimonat-, Tetrafluoroborat-, Tetrachloroferrat-, Hexafluoroarsenat- oder Hexafluorophosphatanion darstellt.

5. Salze nach Anspruch 1, dadurch gekennzeichnet, daß $Z^{t-}$ für $R^9SO_3^-$, worin $R^9$ eine aromatische Gruppe mit 6 bis 12 Kohlenstoffatomen oder eine aliphatische Gruppe mit 1 bis 4 Kohlenstoffatomen bedeutet, oder ein Phosphat- oder Sulfatanion steht.

6. Salze nach Anspruch 1, dadurch gekennzeichnet, daß $R^7$ und $R^8$ für gegebenenfalls durch unter Alkyl- und Alkoxygruppen mit 1 bis 4 Kohlenstoffatomen, Phenylgruppen, Nitrogruppen und Halogenatomen ausgewählte Gruppen einfach oder zweifach substituierte Phenyl- oder Naphtylgruppen stehen.

7. Verfahren zur Herstellung von Diaryljodosylsalzen nach einem der Ansprüche 1 bis 6, dadurch gekennzeichnet, daß man ein Diaryljodosylhydroxyd der Formel

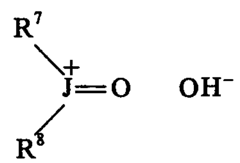 (V)

mit einer Säure der Formel HMXn oder $HR^9SO_3$ oder einer halogenfreien anorganischen Sauerstoffsäure bzw. einem Alkali- oder Ammoniumsalz einer solchen Säure umsetzt, wobei $R^7$ und $R^8$ die in Anspruch 1 oder 6, M, X und n die in einem der Ansprüche 1 bis 4 und $R^9$ die in Anspruch 1 oder 5 angegebenen Bedeutungen haben.

8. Verfahren zur Herstellung von Diaryljodosylsalzen nach einem der Ansprüche 1 bis 6, dadurch gekennzeichnet, daß man ein Diaryljodosylcarbonat der Formel

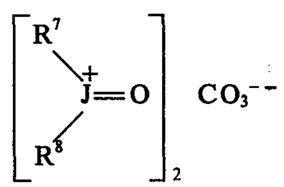 (VI)

mit einer Säure der Formel $HMX_n$ oder $HR^9SO_3$ oder einer halogenfreien anorganischen Sauerstoffsäure umsetzt, wobei $R^7$ und $R^8$ die in Anspruch 1 oder 6, M, X und n die in einem der Ansprüche 1 bis 4 und $R^9$ die in Anspruch 1 oder 5 angegebenen Bedeutungen haben.

9. Verfahren zur Herstellung von Diaryljodosylsalzen nach einem der Ansprüche 1 bis 6, dadurch gekennzeichnet, daß man ein Diaryljodosylacetat der Formel

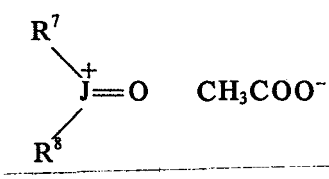 (VII)

ein Diaryljodosyltrifluoracetat der Formel

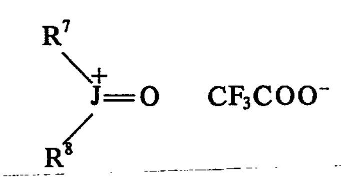 (VIII)

oder ein Diaryljodosylhalogenid der Formel

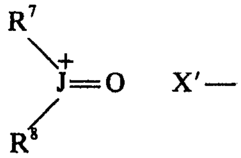

(IX)

mit einem Anion der Formel $Z^{t-}$ umsetzt, wobei $R^7$ und $R^8$ die in Anspruch 1 oder 6 und $Z^{t-}$ die in einem der Ansprüche 1 bis 5 angegebenen Bedeutungen haben und X' ein Halogenatom darstellt.

**Claims**

1. A diaryliodosyl salt of formula

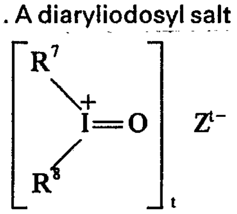

(IV)

wherein $R^7$ and $R^8$ may be the same or different and are each a monovalent aromatic radical of 6 to 22 carbon atoms, $Z^t$ is a t-valent anion of formula $MX_n^-$ or $R^9SO_3^-$ or a t-valent anion of a halogen-free inorganic oxyacid, t is 1, 2 or 3, M is an atom of a metal or metalloid, X is a halogen atom, n is 4, 5, or 6 and is one more than the valency of M, with the proviso that, if M is antimony, n is 6, and if five of the symbols X are each a fluorine atom, then the sixth X is a fluorine atom or a hydroxo group, and $R^9$ is a monovalent aliphatic or aromatic group of 1 to 20 carbon atoms.

2. A salt as claimed in claim 1, in which $Z^t$ is $MX_n^-$, wherein M is an atom of antimony, bismuth, tin, boron, iron, arsenic, or phosphorus.

3. A salt as claimed in either of claims 1 or 2, in which $Z^{t-}$ is $MX_n^-$, wherein X is a fluorine or chlorine atom.

4. A salt as claimed in claim 3, in which $MX_n^-$ is the pentafluorohydroxoantimonate, hexachloroantimonate, hexafluoroantimonate, tetrafluoroborate, tetrachloroferrate, hexafluoroarsenate or hexafluorophosphate, anion.

5. A salt as claimed in claim 1, in which $Z^{t-}$ is $R^9SO_3^-$, wherein $R^9$ is an aromatic group of 6 to 12 carbon atoms or an aliphatic group of 1 to 4 carbon atoms, or a phosphate or sulfate anion.

6. A salt as claimed in claim 1, in which $R^7$ and $R^8$ are phenyl or naphthyl groups which may be unsubstituted or substituted by one or two groups selected from alkyl and alkoxy groups of 1 to 4 carbon atoms, phenyl groups, nitro groups, and halogen atoms.

7. A process for the preparation of a diaryliodosyl salt as claimed in any one of claims 1 to 6, which comprises reaction of a diaryliodosyl hydroxide of formula

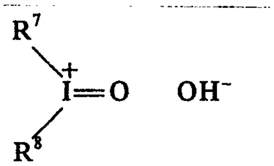

(V)

with an acid of formula $HMX_n$ or $HR^9SO_3$, a halogen-free inorganic oxyacid, or with an alkali metal or ammonium salt of such an acid, wherein $R^7$ and $R^8$ are as defined in either of claims 1 or 6, M, X, and n are as defined in any of claims 1 to 4, and $R^9$ is as defined in either of claims 1 or 5.

8. A process for the preparation of a diaryliodosyl salt as claimed in any of claims 1 to 6, which comprises reaction of a diaryliodosyl carbonate of formula

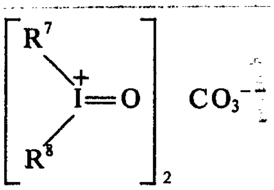

(VI)

with an acid of formula $HMX_n$ or $HR^9SO_3$, or a halogen-free inorganic oxyacid, wherein $R^7$ and $R^8$ are as defined in any of claims 1 to 6, M, X, and n are defined in any of claims 1 to 4, and $R^9$ is as defined in either of claims 1 or 5.

9. A process for the preparation of a diaryliodosyl salt as claimed in any of claims 1 to 6, which comprises reaction of a diaryliodosyl acetate of formula

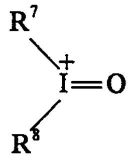 $CH_3COO^-$ (VII)

a diaryliodosyl trifluoroacetate of formula

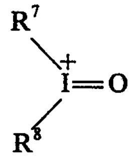 $CF_3COO^-$ (VIII)

or a diaryliodosyl halide of formula

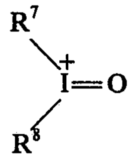 X'— (IX)

with an anion of formula $Z^{t-}$, wherein $R^7$ and $R^8$ are as defined in either of claims 1 or 6, $Z^{t-}$ is as defined in any of claims 1 to 5, and X' is a halogen atom.

**Revendications**

1. Sels de diaryliodosyles répondant à la formule IV:

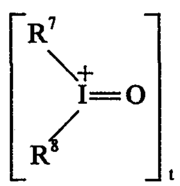 $Z^{t-}$ (IV)

dans laquelle $R^7$ et $R^8$ représentant chacun, indépendamment l'un de l'autre, un radical aromatique univalent contenant de 6 à 22 atomes de carbone, $Z^{t-}$ représente un anion de valence t de formule $MX_n^-$ ou $R^9SO_3^-$ ou un anion de valence t provenant d'un acide oxygéné minéral dépourvu d'halogène, t est égal à 1, à 2 ou à 3, M représente un atome de métal ou de métalloïde, X un atome d'halogène, n est égal à 4, à 5 ou à 6 et est supérieur d'une unité à la valence de M, avec la condition que, lorsque M représente l'antimoine, n soit égal à 6 et que, lorsque 5 des symboles X représentent chacun le fluor, un X représente un atome de fluor ou un radical hydroxo, et $R^9$ représente un radical aliphatique ou aromatique univalent qui contient de 1 à 20 atomes de carbone.

2. Sels selon la revendication 1 caractérisés en ce que $Z^{t-}$ représente $MX_n^-$ où M désigne un atome d'antimoine, de bismuth, d'étain, de bore, de fer, d'arsenic ou de phosphore.

3. Sels selon l'une des revendication 1 et 2, caractérisés en ce que $Z^{t-}$ représente $MX_n^-$ où X désigne un atome de chlore ou de brome.

4. Sels selon la revendication 3 caractérisés en ce que $MX_n^-$ représente l'anion pentafluorohydroxoantimoniate, hexachloro-antimoniate, hexafluoro-antimoniate, tétraflouroborate, tétrachloroferrate, hexafluoro-arséniate ou hexafluorophosphate.

5. Sels selon la revendication 1 caractérisé en ce que $Z^{t-}$ représente $R^9SO_3^-$ où $R^9$ désigne un radical aromatique contenant de 6 à 12 atomes de carbone ou un radical aliphatique contenant de 1 à 4 atomes de carbone, ou encore $Z^{t-}$ désigne un anion phosphate ou sulfate.

6. Sels selon la revendication 1 caractérisé en ce que $R^7$ et $R^8$ représentent chacun un radical phényle ou naphtyle éventuellement porteur d'un ou de 2 substituants pris dans l'ensemble constitué par les radicaux alkyles et alcoxy en $C_1—C_4$, les radicaux phényles, les radicaux nitro et les atomes d'halogènes.

7. Procédé de préparation de sels de diaryliodosyles selon l'une quelconque des revendications 1 à

6, procédé caractérisé en ce qu'on fait réagir un hydroxyde de diaryliodosyle répondant à la formule V:

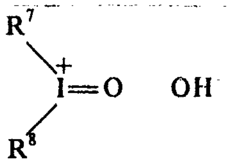

(V)

avec un acide de formule $HMX_n$ un $HR^9SO_3$ ou avec un acide minéral oxygéné dépourvu d'halogène ou un sel de métal alcalin ou d'ammonium d'un tel acide (dans les formules précédentes $R^7$ et $R^8$ ont les significations données à la revendication 1 ou à la revendication 6, M, X et n ont les significations données dans l'une des revendications 1 à 4 et $R^9$ a la signification donnée à la revendication 1 ou 5).

8. Procédé de préparation de sels de diaryliodosyles selon l'une quelconque des revendications 1 à 6, procédé caractérisé en ce qu'on fait réagir un carbonate de diaryliodosyle répondant à la formule VI:

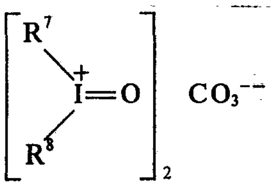

(VI)

avec un acide de formule $HMX_n$ ou $HR^9SO_3$ ou avec un acide oxygéné minéral dépourvu d'halogène (dans ces formules les symboles $R^7$ et $R^8$ ont les significations données dans les revendications 1 et 6, M, X et n ont les significations données dans l'une des revendications 1 à 4 et $R^9$ a la signification donnée à la revendication 1 ou 5).

9. Procédé de préparation de sels de diaryliodosyles selon l'une quelconque des revendications 1 à 6, procédé caractérisé en ce qu'on fait réagir un acétate de diaryliodosyle répondant à la formule VII:

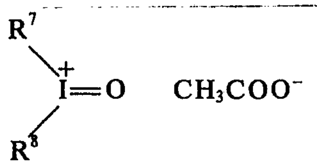

(VII)

ou un trifluoracétate de diaryliodosyle de formule VIII:

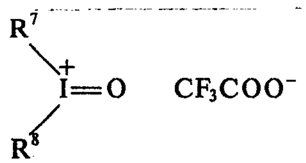

(VIII)

ou un halogénure de diaryliodosyle de formula IX:

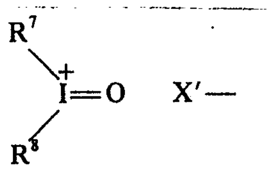

(IX)

avec un anion de formule $Z^{t-}$ (dans les formules précédentes les symboles $R^7$ et $R^8$ ont les significations données dans les revendications 1 et 6, $Z^{t-}$ a la signification donnée dans l'une des revendications 1 à 5 et X' représente un atome d'halogène).